# EUROPEAN PATENT SPECIFICATION

(11) **EP 1 567 667 B1**
(45) Date of publication and mention of the grant of the patent: **15.10.2008**
(21) Application number: 03778516.9
(22) Date of filing: 26.11.2003
(51) Int. Cl.: C12Q 1/68, A61K 31/00, A61P 25/28

(54) **TREATMENT OF HUNTINGTON'S DISEASE WITH EPA**
BEHANDLUNG DER HUNTINGTON'S KRANKHEIT MIT EPA
TRAITEMENT DE LA MALADIE DE HUNTINGTON AU MOYEN D'ACIDE EICOSAPENTAENOIQUE

(30) Priority: 02.12.2002 GB 0228079
(43) Date of publication of application: 31.08.2005
(73) Proprietor: Amarin Neuroscience Limited, Stirling FK7 9JQ (GB)
(72) Inventor: HORROBIN, David Frederick, deceased (GB)
(74) Representative: Wakerley, Helen Rachael
(86) International application number: PCT/GB2003/005131
(87) International publication number: WO 2004/050913

(56) References cited:
- EP-A- 0 614 977
- EP-A- 1 148 873
- WO-A-01/24781
- WO-A-99/15639
- US-A1- 2002 077 361
- US-B1- 6 384 077
- BECHER M W ET AL: "INTRANUCLEAR NEURONAL INCLUSIONS IN HUNTINGTON DISEASE AND DENTATORUBRAL AND PALLIDOLUYSIAN ATROPHY: CORRELATION BETWEEN THE DENSITY OF INCLUSIONS AND IT15 CAG TRIPLET REPEAT LENGTH" NEUROBIOLOGY OF DISEASE, BLACKWELL SCIENTIFIC PUBLICATIONS, OXFORD, GB, vol. 4, no. 6, April 1998 (1998-04), pages 387-397, XP000997534 ISSN: 0969-9961
- NANCE M A: "CLINICAL ASPECTS OF CAG REPEAT DISEASES" BRAIN PATHOLOGY, ZUERICH, CH, vol. 7, no. 3, July 1997 (1997-07), pages 881-900, XP000998301 ISSN: 1015-6305

## Description

Huntington's Disease (HD) is a lethal genetic disease caused by mutations in the gene for the protein Huntingtin on human chromosome 4. The fatty acid, eicosapentaenoic acid (EPA), in any appropriate pharmaceutical form can be used to treat HD (as discussed in European patent application 1148873).

The present invention relates to the treatment of HD and is based on a finding that the therapeutic effect of EPA occurs particularly in those patients with a particular genetic form of HD.

The present invention provides an *in vitro* method of identifying patients with HD, or individuals who are at risk of developing HD, who are particularly likely to respond to treatment with EPA in any appropriate form, wherein of all the fatty acids present in the preparation at least 90% is in the form of EPA and less than 5% is in the form of DHA. The method comprises the steps of carrying out a test to determine the number of CAG repeats in the Huntingtin gene in a sample and identifying those subjects with 45 or fewer repeats as candidates for this treatment.

If the subject has less than 36 repeats, this is an indication of a normal individual. In a preferred test, the subjects selected are those with 44 or fewer, or between 36 and 44, CAG repeats.

The test is carried out on a sample taken from the subject for analysis purposes only and not returned to the subject. The diagnostic step will be in vitro.

The present invention further provides a pharmaceutical composition for treating HD, or for preventing the development of symptoms in individuals who are at risk of developing HD, as set out in the claims. The individuals have 45 or fewer CAG repeats in the gene for huntingtin. Preferably, the subjects selected for administration of the EPA are those with 44 or fewer, or between 36 and 44, CAG repeats.

The EPA used in the present invention is preferably ethyl-EPA.

A CAG repeat number of 46 or more does not show any difference at all on treatment between administration of a placebo and of EPA. In contrast, and unexpectedly, patients suffering from HD who have CAG repeat numbers of 45 or below show a large benefit on administration of EPA.

Although all HD patients have a genetic abnormality in the same gene, not all patients have the same abnormality. The normal gene for huntingtin contains a sequence of CAG repeats which code for a polyglutamine sequence in the gene itself. Even in normal individuals, the polyglutamine sequence is of variable length, but so long as it contains less than 36 CAG repeats and hence less than 36 glutamines in the polyglutamine sequence, the individual will be normal. However, when the sequence contains 36 or more CAG repeats and consequent glutamine sequences, HD will develop. Patients with HD may have anything from 36 to more than 100 CAG repeats.

HD usually starts with movement disorders, particularly affecting the face, head and neck and limbs. These progress and are often accompanied by psychiatric abnormalities and cognitive impairment leading to dementia. The abnormalities are initially caused by huntingtin damage to the neurons of the striatum, but later wide areas of the brain may be involved. Eventually patients become bedridden and completely unable to care for themselves. They usually die 10 to 25 years after the onset of the disease.

The number of CAG repeats has a strong effect on the age of onset of the disease. Patients with numbers only just over 35 may not become ill until their 50s or 60s or even later. Patients with repeat numbers over 60 may become ill in adolescence or even in childhood. Most patients, however, tend to fall ill between the ages of 30 and 50. Once the disease has started, there is a tendency for patients with large numbers of CAG repeats to progress more rapidly although this effect is weak compared to the strong effect on age of onset.

The number of CAG repeats can be identified by diagnostic tests based on the polymerase chain reaction (PCR). These tests provide a firm diagnosis of HD and can, of course, be applied to pre-symptomatic patients. However, relatively few pre-symptomatic individuals who are at risk of being carriers of the HD gene, and therefore who will inevitably develop the disease at some time, bother to get tested. Many people who do have HD symptoms also do not get tested. The main argument for not being tested is that there are no treatments available for HD, so what is the point of knowing exactly that the gene is present and what sort of gene it is.

Clinical trials of the ethyl ester of eicosapentaenoate (ethyl-EPA) in HD have provided strong evidence of the benefit of EPA in HD, and also, completely unexpectedly, of the value of CAG genetic testing.

135 patients with genetically-confirmed HD were entered into a one year trial. They were randomised to receive either 2g/day of ethyl-EPA or an identical-appearing placebo. They were evaluated at baseline, six months and 12 months on the total motor score (TMS) subscale of the Unified Huntington's Disease Rating Scale (UHDRS). The UHDRS is the standard rating scale which is used to monitor the development of HD. The TMS is the component of the UHDRS which changes most reliably, rapidly and consistently and is therefore appropriate for monitoring the outcome of clinical trials.

At the end of one year, change in TMS was compared in the placebo group and the ethyl-EPA group. Overall there was a better outcome on ethyl-EPA than on placebo but this was not statistically significant. However, when patients were stratified on the basis of their CAG repeat numbers, a dramatic benefit of ethyl-EPA was uncovered. Patients who had CAG repeat number of 46 or more did not show any difference at all between placebo and ethyl-EPA. In contrast, patients who had CAG repeat numbers of below 45 showed a large benefit from ethyl-EPA. Placebo patients with CAG repeat numbers 45 and below deteriorated by an average of 5.3%. In contrast, the same group of patients on ethyl-EPA improved over the year by 19.3%. This difference was highly statistically significant on either analysis of covariance or on chi square testing. Particularly striking is the fact that the great majority of patients on ethyl-EPA actually improved. Previously the best that had been hoped for in neurodegenerative diseases like HD was a slowing of deterioration rather than any actual improvement. Since the ethyl-EPA group improved more than three and a half times more than the patients on placebo over one year, this means that after one year the EPA and placebo patients had separated by more than four and a half years of disease progression. Putting it another way, the treated patients had gained at least four and a half years of useful life. In contrast, the patients who had 46 or more CAG repeats did not show any difference between the ethyl-EPA and placebo treatment.

It is preferred that in the methods of
the disclosing,
the EPA treatment is of the nature discussed in European patent application 1148873.

It is preferred to use pure or nearly pure EPA and EPA derivatives. DHA and related fatty acids may not only be ineffective but may actually reduce the efficacy of EPA and its derivatives.

The preparations comprise EPA in an appropriately assimilable form where of all the fatty acids present in the preparation at least 90%, and preferably at least 95%, is in the form of EPA and where less than 5%, and preferably less than 3%, is in the form of docosahexaenoic acid (DHA).

Preferably, among the other fatty acids present there are less than 5%, and preferably less than 3%, of each of AA or DPA-n-3, individually. The same preferably applies for any other fatty acids which might compete with the EPA.

It is preferred that the aggregate DHA, AA and/or DPA-n-3 content is less than 10%, of the total fatty acids present, and preferably less than 5%.

The EPA may be in the form of ethyl-EPA, lithium EPA, mono-, di- or triglyceride EPA or any other ester or salt of EPA, or the free acid form of EPA. The EPA may also be in the form of a 2-substituted derivative or other derivative which slows down its rate of oxidation but does not otherwise change its biological action on psychiatric or brain disorders to any substantial degree (N. Willumsen et al., Biochimica Biophysica Acta, 1998, 1369: 193-203).

The EPA may be combined with a drug which acts primarily on neurotransmitter metabolism or receptors. Suitable drugs for co-administration with the EPA preparations are clozapine; and any one of the class of typical or atypical neuroleptics, including chlorpromazine, haloperidol, risperidone, olanzapine, sertindole, ziprasidone, zotepine or amisulpiride. Standard anti-schizophrenic drugs, antidepressants, tranquillizers, and anti-epileptic drugs, which are used to relieve some of the symptoms of Huntington's disease, may be administered together with the EPA formulations.

As an example of the treatment of Huntington's disease, taken from EP application 1148873, a randomised trial of 96% pure ethyl-EPA was set up in seven severely disabled patients in the final stages of Huntington's disease. All required 24 hour nursing care, had severe movement disorders, were irritable and were partially demented. They were randomised on a double blind basis to receive 2g/day ethyl-EPA or 2g/day placebo for 6 months. During the 6 month period, four patients showed progressive deterioration while three patients reversed the course of the disease and showed improvement with reduced abnormal movements, reduced emotional lability and irritability and improved memory and cognitive function. When the code was broken all four patients who deteriorated were found to be on placebo, while all three patients who improved were found to be taking ethyl-EPA. In four of the patients, two on ethyl-EPA and two on placebo, the brain degeneration was assessed at the beginning and end of the study by magnetic resonance imaging (MRI). MRI allows an accurate assessment of the size of the lateral ventricles, the fluid-filled spaces within the cerebral hemispheres. As Huntington's disease progresses, the lateral ventricles enlarge indicating loss of brain tissue. In the two patients on placebo over 6 months the ventricles enlarged as expected. In the two patients on ethyl-EPA, the MRI showed a reduction in lateral ventricle size indicating an actual reversal of brain tissue loss.

These dramatic results in patients in the end stage of a previously untreatable disease caused by abnormal protein accumulation demonstrate the value of ethyl-EPA in neurodegenerative disorders.

The present invention provides a significant advance in identifying which patients are likely to respond to such a treatment by analysis of the Huntingtin gene. The invention offers the advantage that it is possible to identify patients who are at risk of developing the disease and to administer to them EPA to prevent or postpone the development of symptoms of the disease.

The EPA formulations, in 90% and preferably 95% or even purer forms, may all be administered orally via delivery systems known to those skilled in the art, including soft and hard gelatin capsules; microcapsules in powder, tableted or capsule form; tablets for the solid compound, lithium-EPA; or emulsions made with appropriate natural or synthetic emulsifying agents, including phospholipids or galactolipids. The compounds may also be administered parenterally, either directly, or formulated in various oils or in emulsions or dispersions, using either intravenous, intraperitoneal, intramuscular or sub-cutaneous routes. Topical applications using patch technology or vaginal or rectal forms of application are within the range of the invention.

When combined with a drug used to ease the symptoms of Huntington's, the EPA compound and the other drug may be administered separately, each in their own formulation. They may be packaged separately or be present in the same overall package. Alternatively, using techniques well known to those skilled in the art, the EPA and other drug may be formulated together, so that a daily dose of EPA of 0.1g to 10g per day, and preferably of 0.5g to 5g per day, is provided with the normal daily dose of the other drug.

When supplied alone, the useful daily dose of EPA may be in the range of 0.05g to 50g/day, preferably 0.1g to 10g/day and very preferably 0.5g to 5g/day.

### EXAMPLE FORMULATIONS

In each of the following examples the product is at least 90% and preferably 95% or more pure. This is very important as other fatty acids will compete with the EPA for the binding sites and reduce its efficacy. In particular, fatty acids such as DHA, AA, DPA-n-3 will, individually, be present in concentrations of less than 5% and preferably less than 3%. The total aggregate of such competing compounds must be less than 10% and preferably less than 5%. This degree of purity is also valuable in minimising the volume of material which must be consumed each day, a major factor in helping compliance in psychiatric patients where lack of compliance is a serious problem.
1. Capsules made of hard or soft gelatin which contain 250mg, 500mg, or 1000mg of ethyl-EPA, triglyceride EPA or other appropriate form of EPA.
2. Tablets containing 250mg, 500mg or 1000mg lithium-EPA or hard gelatin capsules containing similar amounts.
3. Emulsions, solutions or dispersions in which the lithium-EPA, ethyl-EPA, triglyceride EPA or other appropriate form of EPA are prepared in a palatable liquid form for oral administration.
4. Suppositories or pessaries into which 100mg to 5g of one of the EPA compounds are formulated.
5. Intravenous solutions or emulsions containing from 10mg to 500mg/ml of one of the EPA compounds.
6-10. As examples 1-5, but using 2-substituted derivatives of EPA.
11-20. As in 1-10 but in which the EPA compound is formulated with the usual dose of any other drug used for the treatment of the symptoms of Huntington's disease.

## Claims

1. An in vitro method of identifying patients with Huntington's disease or individuals who are at risk of developing Huntington's disease, who will respond to treatment comprising administration of an eicosapentaenoic acid (EPA) preparation in any bioavailable form wherein of all the fatty acids present in the preparation at least 90% is in the form of EPA and less than 5% is in the form of docosahexaenoic acid (DHA), comprising the steps of
(a) determining for a sample that has been removed from a subject the number of CAG repeats in the Huntingtin gene; and
(b) identifying those subjects with 45 or fewer repeats as candidates for said treatment.

2. A method according to claim 1, in which the treatment comprises administration of ethyl-EPA.

3. Use of eicosapentaenoic acid (EPA) in any bioavailable form, wherein of all the fatty acids present in the preparation at least 90% is in the form of EPA and less than 5% is in the form of docosahexaenoic acid (DHA), in the manufacture of a medicament for the treatment of Huntington's disease in patients having 45 or fewer CAG repeats in the gene for huntingtin.

4. Use of eicosapentaenoic acid (EPA) in any bioavailable form, wherein of all the fatty acids present in the preparation at least 90% is in the form of EPA and less than 5% is in the form of docosahexaenoic acid (DHA), in the manufacture of a medicament for preventing the development of symptoms in individuals who are at risk of developing Huntington's disease and who have 45 or fewer CAG repeats in the gene for huntingtin.

5. Use according to claim 3 or 4 in which the EPA is in the form of ethyl-EPA.

6. A pharmaceutical composition comprising eicosapentaenoic acid (EPA) in any bioavailable form, wherein of all the fatty acids present in the preparation at least 90% is in the form of EPA and less than 5% is in the form of docosahexaenoic acid (DHA), for the treatment of Huntington's disease in patients having 45 or fewer CAG repeats in the gene for huntingtin.

7. A pharmaceutical composition comprising eicosapentaenoic acid (EPA) in any bioavailable form, wherein of all the fatty acids present in the preparation at least 90% is in the form of EPA and less than 5% is in the form of docosahexaenoic acid (DHA), for preventing the development of symptoms in individuals who are at risk of developing Huntington's disease and who have 45 or fewer CAG repeats in the gene for huntingtin.

8. A pharmaceutical composition according to claim 6 or 7 in which the EPA is in the form of ethyl-EPA.

## Patentansprüche

1. In-vitro-Verfahren zum Identifizieren von Patienten mit der Huntington-Krankheit oder von Personen, die gefährdet sind, an der Huntington-Krankheit zu erkranken, die auf eine Behandlung ansprechen werden, die die Verabreichung eines Eicosapentaensäure-(EPA)-Präparats in einer beliebigen bioverfügbaren Form beinhaltet, wobei von allen Fettsäuren, die in dem Präparat vorliegen, wenigstens 90 % in Form von EPA und weniger als 5 % in Form von Docosahexaensäure (DHA) vorliegen, wobei das Verfahren die folgenden Schritte beinhaltet:
(a) Ermitteln an einer Probe, die von einer Person genommen wurde, der Anzahl von CAG-Wiederholungen im Huntingtin-Gen; und
(b) Bestimmen von Personen mit 45 oder weniger Wiederholungen als Kandidaten für die genannte Behandlung.

2. Verfahren nach Anspruch 1, wobei die Behandlung das Verabreichen von Ethyl-EPA beinhaltet.

3. Verwendung von Eicosapentaensäure (EPA) in einer beliebigen bioverfügbaren Form, wobei von allen Fettsäuren, die in dem Präparat vorliegen, wenigstens 90 % in Form von EPA und weniger als 5 % in Form von Docosahexaensäure (DHA) vorliegen, bei der Herstellung eines Medikaments zur Behandlung der Huntington-Krankheit bei Patienten mit 45 oder weniger CAG-Wiederholungen im Huntingtin-Gen.

4. Verwendung von Eicosapentaensäure (EPA) in einer beliebigen bioverfügbaren Form, wobei von allen Fettsäuren, die in dem Präparat vorliegen, wenigstens 90 % in Form von EPA und weniger als 5 % in Form von Docosahexaensäure (DHA) vorliegen, bei der Herstellung eines Medikaments zur Vorbeugung gegen die Entwicklung von Symptomen bei Personen, die gefährdet sind, an der Huntington-Krankheit zu erkranken, und die 45 oder weniger CAG-Wiederholungen im Huntingtin-Gen haben.

5. Verwendung nach Anspruch 3 oder 4, wobei die EPA in Form von Ethyl-EPA vorliegt.

6. Pharmazeutische Zusammensetzung, die Eicosapentaensäure (EPA) in einer beliebigen bioverfügbaren Form umfasst, wobei von allen Fettsäuren, die in dem Präparat vorliegen, wenigstens 90 % in Form von EPA und weniger als 5 % in Form von Docosahexaensäure (DHA) vorliegen, zur Behandlung der Huntington-Krankheit bei Patienten mit 45 oder weniger CAG-Wiederholungen im Huntingtin-Gen.

7. Pharmazeutische Zusammensetzung, die Eicosapentaensäure (EPA) in einer beliebigen bioverfügbaren Form umfasst, wobei von allen Fettsäuren, die in dem Präparat vorliegen, wenigstens 90 % in Form von EPA und weniger als 5 % in Form von Docosahexaensäure (DHA) vorliegen, zur Vorbeugung gegen die Entwicklung von Symptomen bei Personen, die gefährdet sind, an der Huntington-Krankheit zu erkranken, und die 45 oder weniger CAG-Wiederholungen im Huntingtin-Gen haben.

8. Pharmazeutische Zusammensetzung nach Anspruch 6 oder 7, wobei die EPA in Form von Ethyl-EPA vorliegt.

## Revendications

1. Procédé *in vitro* d'identification de patients atteints de la maladie d'Huntington ou d'individus qui présentent un risque de développer la maladie d'Huntington, qui répondront à un traitement comprenant l'administration d'une préparation d'acide eicopentaénoïque (EPA) dans n'importe quelle forme biodisponible dans lequel de tous les acides gras présents dans la préparation au moins 90% le sont sous forme d'EPA et moins de 5% le sont sous forme d'acide docosahexaénoïque (DHA), comprenant les étapes de
(a) détermination pour un échantillon qui a été prélevé sur un sujet du nombre de répétitions CAG dans le gène Huntingtin ; et
(b) identification des sujets présentant 45 répétitions ou moins comme candidats audit traitement.

2. Procédé selon la revendication 1, dans lequel le traitement comprend l'administration d'éthyl-EPA.

3. Utilisation d'acide eicopentaénoïque (EPA) dans n'importe quelle forme biodisponible, dans laquelle de tous les acides gras présents dans la préparation au moins 90% le sont sous forme d'EPA et moins de 5% le sont sous forme d'acide docosahexaénoïque (DHA), dans la fabrication d'un médicament pour le traitement de la maladie de Huntington chez les patients présentant 45 répétitions CAG ou moins dans le gène Huntingtin.

4. Utilisation d'acide eicopentaénoïque (EPA) dans n'importe quelle forme biodisponible, dans laquelle de tous les acides gras présents dans la préparation au moins 90% le sont sous forme d'EPA et moins de 5% le sont sous forme d'acide docosahexaénoïque (DHA), dans la fabrication d'un médicament pour empêcher le développement de symptômes chez des individus qui présentent un risque de développer la maladie de Huntington et qui présentent 45 répétitions CAG ou moins dans le gène Huntingtin.

5. Utilisation selon la revendication 3 ou 4, dans laquelle l'EPA se présente sous forme d'éthyl-EPA.

6. Composition pharmaceutique comprenant de l'acide eicopentaénoïque (EPA) dans n'importe quelle forme biodisponible, dans laquelle de tous les acides gras présents dans la préparation au moins 90% le sont sous forme d'EPA et moins de 5% le sont sous forme d'acide docosahexaénoïque (DHA), pour le traitement de la maladie de Huntington chez les patients présentant 45 répétitions CAG ou moins dans le gène Huntingtin.

7. Composition pharmaceutique comprenant de l'acide eicopentaénoïque (EPA) dans n'importe quelle forme biodisponible, dans laquelle de tous les acides gras présents dans la préparation au moins 90% le sont sous forme d'EPA et moins de 5% le sont sous forme d'acide docosahexaénoïque (DHA), pour empêcher le développement de symptômes chez des individus présentant un risque de développer la maladie de Huntington et qui présentent 45 répétitions CAG ou moins dans le gène Huntingtin.

8. Composition pharmaceutique selon la revendication 6 ou 7, dans laquelle l'EPA se présente sous forme d'éthyl-EPA.
